**Europäisches Patentamt**

**European Patent Office**

**Office européen des brevets**

(19)

(11) Veröffentlichungsnummer : **0 326 034 B1**

(12)

# EUROPÄISCHE PATENTSCHRIFT

(45) Veröffentlichungstag der Patentschrift :
**26.08.92 Patentblatt 92/35**

(51) Int. Cl.$^5$ : **A61K 35/78, A61K 31/495**

(21) Anmeldenummer : **89100984.7**

(22) Anmeldetag : **20.01.89**

(54) **Kombinationspräparat zur Behandlung von Nervenzell-und Nervenfasererkrankungen und Verletzungen.**

(30) Priorität : **28.01.88 DE 3802567**
**01.02.88 DE 3802895**
**21.04.88 DE 3813451**

(43) Veröffentlichungstag der Anmeldung :
**02.08.89 Patentblatt 89/31**

(45) Bekanntmachung des Hinweises auf die
Patenterteilung :
**26.08.92 Patentblatt 92/35**

(84) Benannte Vertragsstaaten :
**AT BE CH DE ES FR GB GR IT LI LU NL SE**

(56) Entgegenhaltungen :
**EP-A- 0 086 315**
**EP-A- 0 143 977**

(73) Patentinhaber : **Költringer, Peter, Dr.**
**Lortzinggasse 20**
**A-8041 Graz (AT)**

(72) Erfinder : **Költringer, Peter, Dr.**
**Lortzinggasse 20**
**A-8041 Graz (AT)**

(74) Vertreter : **Dipl.-Phys.Dr. Manitz**
**Dipl.-Ing.Dipl.-Wirtsch.-Ing. Finsterwald**
**Dipl.-Phys. Rotermund Dipl.-Chem.Dr. Heyn**
**B.Sc.(Phys.) Morgan**
**Robert-Koch-Strasse 1**
**W-8000 München 22 (DE)**

EP 0 326 034 B1

## Beschreibung

Die vorliegende Erfindung betrifft ein Kombinationspräparat, das sich insbesondere zur Behandlung von Nervenzell- und Nervenfasererkrankungen und Durchblutungsstörungen sowie zur Prophylaxe von Durchblutungsstörungen eignet.

Solche Nervenzell- bzw. Nervenfasererkrankungen umfassen verschiedene Indikationen, z.B.

a) sämtliche Neuropathien,

b) degenerative Nervenfaser- und Nervenzellprozesse im peripheren und zentralen Nervensystem,

c) sämtliche Neuralgien unterschiedlichster Genese,

d) virale und bakterielle Infektionen mit Nervenfaser- und Nervenzellbefall, z.B. Herpes, insbesondere Herpes zoster.

Weiterhin eignet sich das Präparat für Nervenzell- und Nervenfaserregeneration nach Verletzung derselben, und zwar für Nervenfasern aller Qualitäten, d.h. sowohl motorisch als auch sensibel als auch vegetativ.

Viele der oben angegebenen Erkrankungen sind mit extremen und unangenehmen Schmerzen verbunden und lassen sich derzeit nur schlecht oder garnicht behandeln. Auch in Fällen, wo eine gewisse Linderung möglich ist, müssen häufig Medikamente eingesetzt werden, welche unerwünschte Gegenindikationen aufweisen.

Die Aufgabe der vorliegenden Erfindung ist es, hier Abhilfe zu schaffen und ein Kombinationspräparat vorzusehen, das bei den oben erwähnten Erkrankungen bzw. Verletzungen eine weitgehende Linderung von Schmerzen und/oder eine Regeneration der betroffenen Nervenzellen und -fasern bewirkt und das ggf. auch für die Behandlung bzw. Prophylaxe von Durchblutungsstörungen einsetzbar ist.

Zur Lösung dieser Aufgabe sieht die Erfindung ein Kombinationspräparat vor, das zumindest aus Wirkstoffen aus Extr. Ginkgo bilobae (Ginkgoflavonglykoside) und mindestens einer Substanz der Gruppe Folsäure und deren Abkömmlinge, insbesondere Folinsäure; Thioctsäure und deren Abkömmlinge; und sämtliche Vitamin-B-Gruppen und deren Abkömmlinge besteht.

Extr. Ginkgo bilobae, das aus Ginkgoflavonglykoside besteht, ist ein rein pflanzliches Extrakt, das im Handel unter dem Warenzeichen Tebonin (eingetragenes Warenzeichen der Firma Dr. Willmar Schwabe, Karlsruhe) erhältlich ist. Eine Ampulle dieses Produkts zu 5 ml enthält:

Extr. Ginkgo bilbae e fol.sicc. puriss. pro inject. 17,5 mg, stand. auf 4,2 mg Ginkgoflavonglykoside. Tebonin wird bisher in der Praxis für die Behandlung von Durchblutungsstörungen erfolgreich eingesetzt.

Bei der Behandlung von Zuckerkranken mit Veränderungen an den Nerven, d.h. bei der Behandlung von Patienten mit Polyneuropathiesymptom, hat sich überraschenderweise gezeigt, daß die Behandlung mit Tebonin in Kombination mit Folsäure oder mit Thioctsäure zu einer weitgehenden Linderung der Schmerzen und zu einer deutlichen Besserung geführt hat. Dies ist umso erstaunlicher als Folsäure und Thioctsäure normalerweise nicht bei Diabetikern eingesetzt werden, sondern als ein Antidot des in der Tumorchemotherapie verwendeten Folsäure-Antagonisten Methotrexat bzw. für die Behandlung von Hepatitis und akuten Schwermetall vergiftungen.

Aufgrund der bisher durchgeführten klinischen Untersuchungen gibt es Indizien dafür, daß sich das erfindungsgemäße Kombinationspräparat für die Behandlung sämtlicher oben bezeichneter Erkrankungen und Verletzungen einsetzen läßt. Weiterhin ist aufgrund der verwandten Strukturen davon auszugehen, daß die erzielte Wirkung sich auch mit Abkömmlingen bzw. Derivaten der Folsäure und der Thioctsäure sowie mit den genannten Vitaminen der Vitamin-B-Gruppe erreichen läßt.

Weiterhin ist überraschenderweise festgestellt worden, daß das erfindungsgemäße Präparat zumindest in Form einer Kombination von Wirkstoffen Extr. Ginkgo bilobae (Ginkgoflavonglykoside) und einer Substanz oder mehrerer Substanzen der Gruppe Folsäure und deren Abkömmlinge; insbesondere Folinsäure, eine deutlich bessere Behandlung von Durchblutungsstörungen ermöglicht sowie zur Prophylaxe von Durchblutungsstörungen geeignet ist. Anfängliche klinische Untersuchungen zeigen, daß während Tebonin allein eine gute Erniedrigung der Viskoelastizität des Blutes, jedoch keine an sich erwünschte verminderte Trombozytenaggregation bewirken, das Kombinationspräparat im Vergleich dazu eine deutlich weiter verminderte Viskoelastizität sowie eine massive Herabsetzung der Trombozytenaggregation erbringt. Es ist daher anzunehmen, daß das Kombinationspräparat als vorbeugende Maßnahme gegen Herzinfarkte u.a., sich als sehr wirksam erweisen wird. Berücksichtigt man, daß die bisherige Vorbeugung von zu Herzinfarkten führenden Durchblutungsstörungen mit aspirinhaltigen Mitteln erfolgt ist, wobei die Einnahme dieser Mittel zu störenden Nebenwirkungen führt, während das erfindungsgemäße Kombinationspräparat bislang keine unerwünscht Nebenwirkungen gezeigt hat, so wird die enorme Bedeutung des neuen Kombinationspräparats deutlich. Es soll an dieser Stelle kurz erwähnt werden, daß weder Folsäure noch Folinsäure alleine eine Verminderung der Viskosität des Blutes noch eine Senkung der Trombozytenaggregation gezeigt hat. Bei den angestellten Vergleichen ist die Trombozytenaggregation nach vier verschiedenen Kriterien ausgewertet worden und zwar spontan, nach Zusatz von Adenosin-diphosphat, nach Zusatz von Ephinephrin und nach Zusatz von Collagen und daß die oben angegebene Beurteilung nach der Messung der Trombozytenaggregation nach allen vier Kriterien bestätigt worden ist.

Besonders wirksame Kombinationen und Bereiche für anzuwendende Tagesdosen sind den Unteransprüchen zu entnehmen.

Die Erfindung wird nachfolgend näher erläutert anhand von Beispielen.

Beispiel 1

Eine Infusionslösung für eine Tagesdosis ist vorbereitet worden, und zwar aus 250 ml physiologischer Kochsalzlösung mit 25 ml Tebonin und einer 1 ml Ampulle Calciumfolinat der Firma Ebewe, welche 3,24 mg Calciumfolinat (wasserfrei) entsprechend 3 mg Folinsäure in wässriger Lösung enthält.

Drei Patienten mit Diabetes melitus und hyperpathischen Polyneuropathiesyndrom sind dann mit dieser Lösung behandelt worden. Bereits nach 3 Tagen zeichnet sich bei allen Patienten eine deutliche Besserung ab. Die Behandlung ist in allen Fällen solange fortgeführt worden, bis die Patienten schmerzfrei waren. Im günstigsten Fall ist dies bei einem Patienten bereits nach 4 Tagen eingetreten. Bei den anderen Patienten ab dem 11. und 14. Tag. Danach ist die Behandlung abgebrochen worden. Die Zeitdauer für die Infusionen betrug bei allen Patienten zwischen 1 und 2 Stunden pro Tag. Mehr als drei Monate nach Durchführung dieser Behandlung ist bei allen Patienten keine Wiederkehr der Symptome festgestellt worden.

Messungen der Nervenleitgeschwindigkeit haben gezeigt, daß sich diese bereits nach 4 - 8-tägiger Behandlung um 20 bis 30% gebessert hat.

Beispiel 2

Auch hier ist eine Infusionslösung vorbereitet worden, wobei eine Tagesdosis aus 250 ml physiologischer Kochsalzlösung, 25 ml Tebonin und zwei Tioctan-Ampullen bestand, wobei jede Ampulle 5 ml = 50 mg Thioctsäure (6,8-Dithiooctansäure) enthielt. Zwei weitere Patienten mit Diabetes melitus und hyperpathischen Polyneuropathiesyndrom sind mit dieser Lösung behandelt worden, wobei auch hier die Infusion einmal am Tag stattfand mit einer Infusionsdauer zwischen 1 und 2 Stunden.

Auch hier zeichnet sich nach wenigen Tagen eine deutliche Besserung ab und die Behandlung war so erfolgreich, daß sie nach 10 bzw. 14 Tagen abgebrochen werden konnte. Auch nach dieser Behandlung traten nach mehr als einem Monat keine wiederkehrenden Symptome auf.

Bei allen Patienten, die nach den Beispielen 1 und 2 behandelt worden sind, war die Hyperpathie verschwunden. Bei einem Patienten ist eine klinische Tiefensensibilitätsuntersuchung durchgeführt worden und das Ergebnis dieser Untersuchung bestätigt den erreichten Erfolg. Bei keinem der untersuchten Patienten war eine Unverträglichkeitsreaktion festzustellen.

Beispiel 3 - Kontrollbeispiel

Weitere Patienten mit Diabetes melitus und hyperpathischem Polyneuropathiesyndrom sind mit Infusionslösungen behandelt worden, wobei die Lösung wiederum aus physiologischer Kochsalzlösung 250 ml bestand, jedoch jeweils nur mit einem der genannten Wirkstoffe, d.h. Tebonin, Folinsäure und Thioctsäure versehen war.

Bei den Patienten, die mit der Teboninlösung allein behandelt worden sind, erstreckte sich die Behandlungsdauer über 14 Tage, ohne daß eine Besserung des Zustands überhaupt eintrat. Auch bei den Patienten, die mit der Folinsäurelösung behandelt worden sind, war nach 14 Tagen keine Besserung festzustellen. Die Patienten, die mit der Thioctsäurelösung behandelt worden sind, sind sogar bis zu drei Wochen lang behandelt worden, ohne daß eine bemerkbare Wirkung eingetreten ist.

Beispiel 4

Ein Patient mit Carpaltunnelsyndrom ist mit der Infusionslösung gemäß Beispiel 1 behandelt worden. Nach einer Behandlungsdauer von einer Woche war bei diesem Patienten bereits ein deutlicher Rückgang der Beschwerden festzustellen.

Die Behandlung wird derzeit fortgeführt und der Zustand des Patienten verbessert sich von Tag zu Tag.

Beispiel 5

Nachdem sich das Kombinationspräparat bei der Behandlung von Nervenzell- und Nervenfasererkrankungen als sehr wirksam erwiesen hat, ist bei 2 Patienten untersucht worden, ob das Präparat bei der Behandlung von Durchblutungsstörungen im Vergleich zu der Behandlung von Tebonin allein eine verbesserte Wirkung bringt. Bei dieser Untersuchung ist festgestellt worden, daß das Kombinationspräparat zu einer Verminderung der Viskosität und einer Verbesserung der Aggregationseigenschaften des Blutes der Patienten führte, wobei diese Veränderung länger angehalten hat und mit geringeren Dosen erreichbar war als bei der Behandlung mit Tebonin allein. Aufgrund dieser anfänglichen Ergebnisse vermutet man, daß sich das Kombinationspräparat bestens für die Behandlung von sämtlichen Durchblutungsstörungen eignet, sowohl periphäre als auch zentrale Durchblutungsstörungen.

Beispiel 6

Um die am Ende des Beispiels 5 geäußerte Vermutung zu belegen und um festzustellen, ob eine Vorbeugung von Durchblutungsstörungen möglich wäre, sind 10 freiwillige gesunde Patienten, teils männlich, teils weiblich im Alter von 20 bis 40 Jahren über einen Zeitraum von 10 Tagen täglich mit dem Kombinationspräparat behandelt worden. In diesem Fall ist das Kombinationspräparat in Pillenform verabreicht worden, wobei jeder Patient täglich auf einmal zwei Tabletten Tebonin (jede Tablette enthält: Trockenextrakt aus Ginkgo-biloba-Blättern (50:1) 40 mg eingestellt auf 9,6 mg Ginkgoflavonglykoside) und 1 Tablette Folsan (eingetragenes Warenzeichen) welche 5 mg Folsäure (Aktivteil Folinsäure) enthält, eingenommen hat.

Zur Kontrolle sind weitere Patienten mit Tebonin allein und noch weitere mit Folsäure allein behandelt worden. Blutproben von den Patienten sind dann im Hinblick auf die Viskoelastizität und Trombozytenaggregation untersucht worden. Zur Untersuchung der Trombozytenaggregation ist ein Teil der vorgenommenen Blutproben in vier Proben geteilt worden und diese vier Proben sind jeweils nach den vier üblichen Kriterien der Trombozytenaggregation untersucht worden, d.h. spontan, nach Zusatz von Adenosin-diphosphat, nach Zusatz von Ephinephrin bzw. nach Zusatz von Collagen untersucht worden.

Nach statistischer Auswertung ist festgestellt worden, daß im Vergleich zu Tebonin allein die Behandlung mit dem erfindungsgemäßen Kombinationspräparat eine deutliche weitere Verminderung der Viskoelastizität sowie eine massive Verminderung der Trombozytenaggregation erreicht worden ist. Bekanntlich ist Tebonin allein nicht in der Lage eine Verminderung der Trombozytenaggregation zu bewirken. Folsäure bewirkt weder eine Verminderung der Viskoelastizität noch eine Senkung der Trombozytenaggregation.

Im Verlauf dieser Untersuchung ist auch festgestellt worden, daß nach peroraler Einnahme von vier Tebonintabletten und einer Folsantablette eine deutliche Besserung der Viskoelastizität sowie der Trombozytenaggregation nach drei Stunden erreicht ist. Somit eignet sich eine solche Behandlung als Sofortmaßnahme nach einem Herzinfarkt.

**Patentansprüche**

1. Kombinationspräparat, insbesondere zur Behandlung von Nervenzell- und Nervenfasererkrankungen und Durchblutungsstörungen sowie zur Prophylaxe von Durchblutungsstörungen bestehend aus zumindest den Wirkstoffen Extr. Ginkgo bilobae (Ginkgoflavonglykoside) und mindestens einer Substanz der Gruppe: Folsäure und deren Abkömmlinge, insbesondere Folinsäure; Thioctsäure und deren Abkömmlinge; und sämtliche Vitamin-B Gruppen und deren Abkömmlinge.

2. Kombinationspräparat nach Anspruch 1, dadurch gekennzeichnet, daß die Wirkstoffe mindestens aus Tebonin (eingetragenes Warenzeichen der Firma Dr. Willmar Schwabe, Karlsruhe) und aus Calciumfolinat (wasserfrei) und/oder Folinsäure bestehen.

3. Kombinationspräparat nach Anspruch 1 oder 2, dadurch gekennzeichnet, daß eine Tagesdosis mindestens aus 5-100 ml Tebonin und 1 bis 15 mg Folinsäure, d.h. etwa 1,08 bis 16,2 mg Calciumfolinat, insbesondere aus 20-35 ml Tebonin und 2 bis 4 mg Folinsäure, d.h. 2,16 bis 4,32 mg Calciumfolinat und vorzugsweise aus 25 ml Tebonin und 3 mg Folinsäure, d.h. 3,24 mg Calciumfolinat besteht.

4. Kombinationspräparat nach Anspruch 1, dadurch gekennzeichnet, daß die Wirkstoffe mindestens aus Tebonin und Thioctsäure bestehen.

5. Kombinationspräparat nach Anspruch 1 oder Anspruch 4, dadurch gekennzeichnet, daß eine Tagesdosis mindestens aus 5 - 100 ml Tebonin und 25 - 400 mg Thioctsäure, insbesondere aus 20 - 35 ml Tebonin und 40 bis 150 mg Thioctsäure, vorzugsweise 25 ml Tebonin und 100 mg Thioctsäure besteht.

6. Kombinationspräparat nach Anspruch 1, dadurch gekennzeichnet, daß die Wirkstoffe mindestens aus Tebonin und ein oder mehrere der Vitamine $B_1$, $B_2$, $B_6$, $B_{12}$ oder einem B-Komplex bestehen.

7. Kombinationspräparat nach Anspruch 1 oder Anspruch 6, dadurch gekennzeichnet, daß eine Tagesdosis mindestens aus 5 bis 100 ml Tebonin und 0,1 bis 0,4 gm krist. Vitamin $B_1$ (Aneurin) besteht.

8. Kombinationspräparat nach Anspruch 1 oder Anspruch 6, dadurch gekennzeichnet, daß eine Tagesdosis mindestens aus 5 bis 100 ml Tebonin und 10 bis 50 mg Vitamin $B_2$ (Lactoflavin) besteht.

9. Kombinationspräparat nach Anspruch 1 oder Anspruch 6, dadurch gekennzeichnet, daß eine Tagesdosis mindestens aus 5 bis 100 ml Tebonin und 20 bis 250 mg Vitamin $B_6$ (Pyridoxin.hydrochlor.) besteht.

10. Kombinationspräparat nach Anspruch 1 oder Anspruch 6, dadurch gekennzeichnet, daß eine Tagesdosis mindestens aus 5 bis 100 ml Tebonin und 0,2 bis 10 mg Vitamin $B_{12}$ (Cyanocobalamin) besteht.

11. Kombinationspräparat nach Anspruch 1 oder Anspruch 6, dadurch gekennzeichnet, daß eine Tagesdosis mindestens aus 5 bis 100 ml Tebonin und Vitamin B-Komplex mit Aneurin 5 bis 20 mg Lactoflavin 1 bis 4 mg Nicotinsäureamid 10 bis 50 mg Adermin 1 bis 9 mg und pantothensaures Calcium 2 bis 20 mg und ggf. Pyridoxin 0,5 bis 4 mg, besteht.

12. Kombinationspräparat nach einem der vorhergehenden Ansprüche, dadurch gekennzeichnet, daß die Wirkstoffe für eine Tagesdosis durch Infusion in einer physiologischen Kochsalzlösung, insbesondere in 250 ml derselben verabreicht werden.

13. Kombinationspräparat nach einem der vorhergehenden Ansprüche, dadurch gekennzeichnet, daß die Wirkstoffe in unterschiedlichen Darbietungsformen vorliegen, insbesondere in Pulverform, ggf. zu Pillen verpreßt, in flüssiger Form, in Ampullen oder als Salbe zur Behandlung von offenen Wunden.

14. Kombinationspräparat nach Anspruch 1, dadurch gekennzeichnet, daß die Wirkstoffe in Pillen-, Flüssig- oder Pulverform zur peroralen Einnahme vorliegen.

15. Kombinationspräparat nach Anspruch 14, insbesondere zur Prophylaxe von Durchblutungsstörungen, dadurch gekennzeichnet, daß eine Tagesdosis aus Trockenextrakt aus Ginkgo-biloba-Blättern (50 : 1) 40 bis 160 mg eingestellt auf 9,6-38,4 mg Ginkgoflavonglykoside und 1 - 15 mg Folsäure, vorzugsweise Ginkgo-biloba-Blättern (50 : 1) 80 bis 120 mg eingestellt auf 19,2 - 28,8 mg Ginkgoflavonglykoside und 3 - 8 mg Folsäure besteht.

16. Kombinationspräparat nach Anspruch 15, dadurch gekennzeichnet, daß eine Tagesdosis aus 1 - 4 Pillen Tebonin (eingetragenes Warenzeichen) und einer Pille Folsan (eingetragenes Warenzeichen), vorzugsweise 2 - 3 Pillen Tebonin und eine Pille Folsan besteht.

## Claims

1. Combination preparation, in particular for the treatment of nerve cell and nerve fibre diseases and of circulatory disturbances, and also for the prophylaxis of circulatory disturbances, the preparation comprising at least the active substances Extr. Ginkgo bilobae (ginkgo flavonglycosides) and at least one substance of the group: folic acid and its derivatives, in particular folinic acid; $\alpha$-lipoic.acid and its derivatives; and all the vitamin-B groups and their derivatives.

2. Combination preparation in accordance with claim 1, characterised in that the active substances comprise at least Tebonin (registered trademark of the company Dr. Willmar Schwabe, Karlsruhe) and calcium folinate (anhydrous) and/or folinic acid.

3. Combination preparation in accordance with claim 1 or claim 2, characterised in that a daily dosage comprises at least 5-100 ml Tebonin and 1 to 15 mg folinic acid, i.e. approximately 1.08 to 16.2 mg calcium folinate, in particular 20-35 ml Tebonin and 2 to 4 mg folinic acid, i.e. 2.16 to 4.32 mg calcium folinate, and preferably of 25 ml Tebonin and 3 mg folinic acid, i.e. 3.24 mg calcium folinate.

4. Combination preparation in accordance with claim 1, characterised in that the active substances comprise at least Tebonin and $\alpha$-lipoic acid.

5. Combination preparation in accordance with claim 1 or claim 4, characterised in that a daily dosage comprises at least 5 - 100 ml Tebonin and 25 to 400 mg $\alpha$-lipoic acid, in particular of 20 - 35 ml Tebonin and 40 to 150 mg $\alpha$-lipoic acid, and preferably 25 ml Tebonin and 100 mg $\alpha$-lipoic acid.

6. Combination preparation in accordance with claim 1, characterised in that the active substances comprise at least Tebonin and one or more of the the vitamins $B_1$, $B_2$, $B_6$, $B_{12}$ or a B-complex.

7. Combination preparation in accordance with claim 1 or claim 6, characterised in that a daily dosage comprises at least 5 to 100 ml Tebonin and 0.1 to 0.4 mg of crystalline vitamin $B_1$ (aneurin).

8. Combination preparation in accordance with claim 1 or claim 6, characterised in that a daily dosage comprises at least 5 to 100 ml Tebonin and 10 to 50 mg vitamin $B_2$ (lactoflavin).

9. Combination preparation in accordance with claim 1 or claim 6, characterised in that a daily dosage comprises at least 5 to 100 ml Tebonin and 20 to 250 mg vitamin $B_6$ (pyridoxin-hydrochloride).

10. Combination preparation in accordance with claim 1 or claim 6, characterised in that a daily dosage comprises at least 5 to 100 ml Tebonin and 0.2 to 10 mg vitamin $B_{12}$ (cyanocobalamin).

11. Combination preparation in accordance with claim 1 or claim 6, characterised in that a daily dosage comprises at least 5 to 100 ml Tebonin and vitamin B-complex with aneurin 5 to 20 mg, lactoflavin 1 to 4 mg, nicotinic acid amide 10 to 50 mg, adermin 1 to 9 mg and pantothenic acid salts of calcium 2 to 20 mg, and optionally pyridoxin 0.5 to 4 mg.

12. Combination preparation in accordance with one of the preceding claims, characterised in that the active substances for a daily dose are administered by infusion of a physiological kitchen salt solution, in particular in 250 ml of the same.

13. Combination preparation in accordance with one of the preceding claims, characterised in that the active substances are present in various administration forms, in particular in powder form, optionally pressed into pills, in liquid form, in ampoules, or as an ointment for treating open wounds.

14. Combination preparation in accordance with claim 1, characterised in that the active substances are present in pill form, in liquid form or in powder form for peroral administration.

15. Combination preparation in accordance with claim 14, in particular for the prophylaxis of circulatory disturbances, characterised in that a daily dosage comprises dried extract of ginkgo-biloba leaves (50 : 1) 40 to 160 mg adjusted to 9.6-38.4 mg ginkgo flavonglycosides and 1 - 15 mg folic acid, preferably ginkgo-biloba leaves (50 : 1) 80 to 120 mg adjusted to 19.2 - 28.8 mg ginkgo flavonglycosides and 3 - 8 mg folic acid.

16. Combination preparation in accordance with claim 15, characterised in that a daily dosage comprises 1 - 4 pills Tebonin (registered trademark) and one pill folsan (registered trademark), preferably 2 - 3 pills Tebonin and one pill Folsan.

## Revendications

1. Préparation combinée, en particulier pour le traitement des maladies des cellules nerveuses et des fibres nerveuses et le traitement d'affections circulatoires ainsi que pour la prophylaxie d'affections circulatoires, comprenant au moins le principe actif extr. ginkgo bilobae (ginkgoflavoneglycoside) et au moins une substance du groupe: acide folique et ses dérivés, en particulier acide folinique; acide $\alpha$-lipoique et ses dérivés; et tous les groupes de vitamines B et leurs dérivés.

2. Préparation composée selon la revendication 1, caractérisée en ce que les substances actives comprennent au moins du Tebonin (marque déposée de la société Dr. Willmar Schwabe, Karlsruhe) et du folinate de calcium (anhydre) et/ou de l'acide folinique.

3. Préparation composée selon l'une ou l'autre des revendications 1 et 2, caractérisée en ce qu'une dose quotidienne comprend au moins 5-100 ml de Tebonin et 1 à 15 mg d'acide folinique, c'est-à-dire environ 1,08 à 16,2 mg de folinate de calcium, en particulier 20-35 ml de Tebonin et 2 à 4 mg d'acide folinique, c'est-à-dire 2,16 à 4,32 mg de folinate de calcium et de préférence 25 ml de Tebonin et 3 mg d'acide folinique, c'est-à-dire 3,24 mg de folinate de calcium.

4. Préparation composée selon la revendication 1, caractérisée en ce que les produits actifs comprennent au moins du Tebonin et de l'acide α-lipoique.

5. Préparation composée selon l'une ou l'autre des revendications 1 et 4, caractérisée en ce qu'une dose quotidienne comprend au moins 5 - 100 ml de Tebonin et 25 - 400 mg d'acide α-lipoique, en particulier 20 - 35 ml de Tebonin et 40 à 150 mg d'acide α-lipoique, de préférence 25 ml de Tebonin et 100 mg d'acide α-lipoique.

6. Préparation composée selon la revendication 1, caractérisée en ce que les produits actifs comprennent au moins du Tebonin et une ou plusieures des vitamines B1, B2, B6, B12, ou un complexe de vitamines B.

7. Préparation composée selon l'une ou l'autre des revendications 1 et 6, caractérisée en ce qu'une dose quotidienne comprend au moins 5 à 100 ml de Tebonin et 0,1 à 0,4 mg de vitamine B1 cristallisée (aneurine).

8. Préparation composée selon l'une ou l'autre des revendications 1 et 6, caractérisée en ce qu'une dose quotidienne comprend au moins 5 à 100 ml de Tebonin et 10 à 50 mg de vitamine B2 (lactoflavine).

9. Préparation composée selon l'une ou l'autre des revendications 1 et 6, caractérisée en ce qu'une dose quotidienne comprend au moins 5 à 100 ml de Tebonin et 20 à 250 mg de vitamine B6 (hydrochlorure de pyridoxine).

10. Préparation composée selon l'une ou l'autre des revendications 1 et 6, caractérisée en ce qu'une dose quotidienne comprend au moins 5 à 100 ml de Tebonin et 0,2 à 10 mg de vitamine B12 (cyanocobalamine).

11. Préparation composée selon l'une ou l'autre des revendications 1 et 6, caractérisée en ce qu'une dose quotidienne comprend au moins 5 à 100 ml de Tebonin et un complexe de vitamines B comprenant 5 à 20 mg d'aneurine, 1 à 4 mg de lactoflavine, 10 à 50 mg d'amide d'acide nicotinique, 1 à 9 mg d'adermine et 2 à 20 mg de sel de calcium d'acide pantothénique, et en option 0,5 à 4 mg de pyridoxine.

12. Préparation composée selon l'une des revendications précédentes, caractérisée en ce que les produits actifs pour une dose quotidienne sont administrés par perfusion dans une solution de chlorure de sodium physiologique, en particulier dans 250 ml de cette solution.

13. Préparation composée selon l'une des revendications précédentes, caractérisée en ce que les produits actifs existent sous des formes galéniques différentes, en particulier sous la forme de poudre, éventuellement comprimée sous la forme de dragées, sous forme liquide, en ampoules ou sous la forme de pommade pour le traitement de blessures ouvertes.

14. Préparation composée selon la revendication 1, caractérisée en ce que les produits actifs existent sous la forme de dragées, sous la forme liquide ou sous la forme de poudre pour administration orale.

15. Préparation composée selon la revendication 14, en particulier pour la prophylaxie d'affections circulatoires, caractérisée en ce qu'une dose quotidienne comprend 40 à 160 mg d'extrait sec de feuilles de ginkgo biloba (50 : 1) dosé à 9,6 - 38,4 mg de ginkgoflavoneglycoside et 1 - 15 mg d'acide folique, de préférence 80 à 120 mg de feuilles de ginkgo biloba (50 : 1) dosés à 19,2 - 28,8 mg de ginkgoflavoneglycoside et 3 - 8 mg d'acide folique.

16. Préparation composée selon la revendication 15, caractérisée en ce qu'une dose quotidienne comprend 1 - 4 dragées de Tebonin (marque déposée) et une dragée de Folsan (marque déposée), de préférence 2 - 3 dragées de Tebonin et une dragée de Folsan.